(19) Europäisches Patentamt
European Patent Office
Office européen des brevets

(11) Veröffentlichungsnummer : **0 165 564 B1**

(12) # EUROPÄISCHE PATENTSCHRIFT

(45) Veröffentlichungstag der Patentschrift : **25.05.88**

(51) Int. Cl.⁴ : **C 07 C103/44**, C 07 C102/00

(21) Anmeldenummer : **85107326.2**

(22) Anmeldetag : **13.06.85**

(54) **Verfahren zur Herstellung von 4-Acylamino-2-alkylamino-phenylethern.**

(30) Priorität : **20.06.84 DE 3422809**
**13.10.84 DE 3437665**

(43) Veröffentlichungstag der Anmeldung :
**27.12.85 Patentblatt 85/52**

(45) Bekanntmachung des Hinweises auf die Patenterteilung : **25.05.88 Patentblatt 88/21**

(84) Benannte Vertragsstaaten :
**BE CH DE FR GB IT LI**

(56) Entgegenhaltungen :
**EP-A- 0 011 048**
**EP-A- 0 096 815**
**JOURNAL OF THE CHEMICAL SOCIETY: "Perkin Transactions II Physical Organic Chemistry", Teil II, Seiten 1442-1448, 1977, London, GB; M.R. CRAMPTON "The stabilities of Meisenheimer Complexes. Part 14. Equilibrium a kinetic data for sodium ethoxide addition to 2,4-dinitro-6-X-pheneto in ethanol"**
**PATENT ABSTRACTS OF JAPAN, Band 5, Nr. 71 (C-54)[743], 13. Mai 1981; & JP - A - 56 18 945 (SUMITOMO KAGAKU KOGYO) 23.02.1981**

(73) Patentinhaber : **HOECHST AKTIENGESELLSCHAFT**
**Postfach 80 03 20**
**D-6230 Frankfurt am Main 80 (DE)**

(72) Erfinder : **Hähnle, Reinhard, Dr.**
**Kastanienweg 7a**
**D-6231 Sulzbach (DE)**
Erfinder : **Hintzmann, Manfred, Dr.**
**Sachsenring 48**
**D-6238 Hofheim am Taunus (DE)**
Erfinder : **Koller, Wolfgang, Dr.**
**Im Brühl 14**
**D-6000 Framkfurt am Main 71 (DE)**
Erfinder : **Papenfuhs, Theodor, Dr.**
**Heinrich-Bleicher-Strasse 40**
**D-6000 Frankfurt am Main 50 (DE)**

**EP 0 165 564 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein Verfahren zur Herstellung von 4-Acylamino-2-alkylamino-phenylethern durch wasserfreie Umsetzung von 2,4-Dinitrochlorbenzol mit Alkalimetallalkoholaten oder -phenolaten in polaren organischen Lösungsmitteln zu den 2,4-Dinitrophenylethern, deren Reduktion zu den 2,4-Diaminophenylethern in Gegenwart von Schwermetallkatalysatoren und katalytischen Mengen von stickstoffhaltigen organischen Basen, deren selektive Acylierung zu den 4-Acylamino-2-amino-phenylethern und deren reduktive Alkylierung mit Aldehyden in Gegenwart von Schwermetallkatalysatoren und katalytischen Mengen organischer stickstoffhaltiger Basen.

Die Herstellung von 4-Acylamino-2-amino-phenylethern aus 2,4-Dinitrochlorbenzol ist im Prinzip literaturbekannt (EP 0 011 048). Bei der dort beschriebenen Umsetzung von 2,4-Dinitrochlorbenzol mit Alkohol/Alkalilauge oder Alkohol/Alkalihydroxid in die entsprechenden 2,4-Dinitrophenylether läßt sich aber die Bildung von 2,4-Dinitrophenol nie völlig ausschließen, wodurch eine aufwendige Abtrennung dieses Nebenproduktes zwingend erforderlich wird, weil seine Gegenwart in den Folgestufen technisch nicht beherrschbare Probleme der Reaktionsführung und Produktqualität aufwirft. Ein angestrebtes Eintopfverfahren auf dieser Basis ist daher nicht realisierbar. Die Reduktion der 2,4-Dinitrophenylether wird nach EP 0 011 048 in einer Wasserstoffatmosphäre in Gegenwart von Edelmetallkatalysatoren ohne Zusatz organischer Stickstoffbasen als Promotoren durchgeführt, wodurch sich die stark exotherme Reaktion nicht zufriedenstellend kontrollieren läßt. Unzureichende Temperaturkontrolle führt aber zur Bildung schwarzer Nebenprodukte, die Qualität und Ausbeute mindern. Bei der Acylierung von 2,4-Diamino-phenylethern (EP 0 011 048) wird nicht nur der gewünschte 4-Acylamino-2-amino-phenylether gebildet, sondern das Reaktionsgemisch enthält daneben immer etwas nicht umgesetztes Ausgangsmaterial und diacylierte Verbindung. Ein ähnliches Selektivitätsproblem konnte bei der reduktiven Alkylierung beispielsweise unter den Bedingungen der DE-OS 2 745 552 oder der Schweizer Patentschrift 310 827 bisher nicht zufriedenstellend gelöst werden.

Es wurde nun überraschenderweise gefunden, daß man 4-Acylamino-2-alkylamino-phenylether der allgemeinen Formel (1)

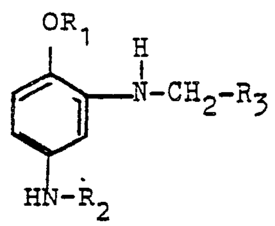

(1)

in welcher $R_1$ eine Alkyl $C_1$-$C_4$ oder Alkoxy $C_1$-$C_4$ alkyl $C_1$-$C_4$ Gruppe oder eine Phenylgruppe, die durch Chlor- oder Bromatome, Alkyl $C_1$-$C_4$, Alkoxy $C_1$-$C_4$, Trifluormethyl-, Carbonsäureamid-, —NH—Alkyl $C_1$-$C_4$, —N(Alkyl $C_1$-$C_4$)$_2$, —$SO_2$—$NH_2$—, —$SO_2$—NH—Alkyl $C_1$-$C_4$, —$SO_2$—N(Alkyl $C_1$-$C_4$)$_2$—, Acetyl-, Benzoylamino- oder —NH—CO—Alkyl $C_1$-$C_4$-Gruppen substituiert sein kann, $R_2$ eine

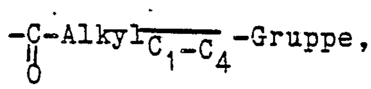

und $R_3$ eine Alkyl $C_1$-$C_4$ Gruppe bedeuten, ausgehend vom 2,4-Dinitrochlorbenzol, in vorteilhafterer Weise und in höheren Ausbeuten herstellen kann, indem man wasserfrei in einem polaren organischen Lösungsmittel 2,4-Dinitrochlorbenzol mit einem Alkalimetallalkoholat oder -phenolat der allgemeinen Formel (2)

$$R_1—OMe \qquad (2)$$

worin $R_1$ die vorstehend genannte Bedeutung hat, und Me ein Alkalimetallatom darstellt, bei Temperaturen von — 30 °C bis 100 °C, vorzugsweise von 0 °C bis 10 °C, ggfs. in Abhängigkeit vom angewandten organischen Lösungsmittel unter Druck, zum 2,4-Dinitrophenyläther der allgemeinen Formel (3)

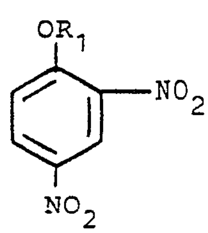

(3)

worin $R_1$ die vorstehend genannte Bedeutung hat, umsetzt, diesen nach oder vorzugsweise ohne Zwischenisolierung in einem polaren organischen Lösungsmittel, vorzugsweise im gleichen Lösungsmittel, in welchem der Chloraustausch erfolgte, in einer Wasserstoffatmosphäre in Gegenwart eines Edelmetallkatalysators der 8. Nebengruppe des Periodischen Systems (Ruthenium bis Platin) und einer katalytischen Menge einer organischen Stickstoffbase (Promotor) bei Temperaturen von 50 bis 120 °C, vorzugsweise von 50-60 °C, ggfs. in Abhängigkeit vom eingesetzten organischen Lösungsmittel unter Druck, zum entsprechenden 2,4-Diaminophenylether reduziert, diesen nach oder vorzugsweise ohne Zwischenisolierung in einem polaren organischen Lösungsmittel, vorzugsweise im gleichen Lösungsmittel, in welchem die Reduktion erfolgte, bei Temperaturen von — 30 bis + 90 °C, vorzugsweise von + 5 bis — 15 °C, ggfs. in Abhängigkeit vom eingesetzten organischen Lösungsmittel unter Druck, selektiv acyliert zum entsprechenden 4-Acylamino-2-amino-phenylether der allgemeinen Formel (4)

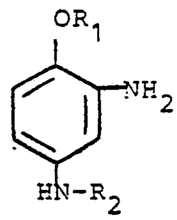

(4)

in welcher $R_1$ und $R_2$ die vorstehend genannten Bedeutungen haben und diesen nach oder vorzugsweise ohne Zwischenisolierung in einem polaren organischen Lösungsmittel mit einem Aldehyd der allgemeinen Formel (5)

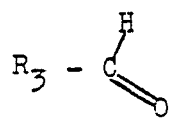

(5)

in welcher $R_3$ die vorstehend genannte Bedeutung hat, in einer Wasserstoffatmosphäre in Gegenwart eines Edelmetallkatalysators der 8. Nebengruppe des Periodischen Systems (Ruthenium bis Platin) und einer katalytischen Menge einer organischen Stickstoffbase (Promotor) bei Temperaturen von 30 bis 120 °C, vorzugsweise von 50-60 °C, ggfs. in Abhängigkeit vom eingesetzten organischen Lösungsmittel unter Druck, selektiv alkyliert zur Verbindung der genannten allgemeinen Formel (1).

Was die einzelnen Stufen hinsichtlich überraschender Ergebnisse anbelangt, so ist es als überraschend zu erachten, daß bei der Umsetzung des 2,4-Dinitrochlorbenzols mit Alkalimetallalkoholat oder -phenolat anstelle von Alkalimetallhydroxid nicht nur die unerwünschte Bildung von 2,4-Dinitrophenol vollständig vermieden wird, sondern daß auch die Ausbeute an 2,4-Diaminophenylether bei der darauf folgenden Reduktion des 2,4-Dinitrophenylethers erheblich verbessert wird.

Bei der katalytischen Reduktion des 2,4-Dinitrophenyläthers ist es als überraschend zu erachten, daß der Zusatz katalytischer Mengen einer organischen Stickstoffbase, wie beispielsweise Triethylamin, einen gleichförmigen Verlauf der exothermen Reaktion gewährleistet, wodurch eine Qualitäts- und Ausbeutesteigerung bewirkt wird.

Was die reduktive Alkylierung des 4-Acylamino-2-amino-phenylethers zum Endprodukt anbelangt, so war es überraschend, daß die Verwendung eines Edelmetallkatalysators in Gegenwart einer katalytischen Menge einer organischen Stickstoffbase, wie beispielsweise eines niederen Trialkylamins, einen höheren Umsatz, eine bessere Selektivität und eine höhere Ausbeute bewirkt.

Hinsichtlich Durchführung des Verfahrens sei im einzelnen folgendes dargelegt:

Es ist zweckmäßig, zur Lösung des 2,4-Dinitrochlorbenzols in einem polaren organischen Lösungsmittel, wie beispielsweise in einem geradkettigen oder verzweigten Alkanol von 1-4 Kohlenstoffatomen, beispielsweise Methanol oder Äthanol, oder in einem geradkettigen oder verzweigten Alkyl $C_1$-$C_4$ acetat, beispielsweise Äthylacetat, Propylacetat n-Butylacetat oder iso-Butylacetat, oder in einem Glykol oder Glykoläther, wie beispielsweise Diäthylenglykol oder Methylglykol, eine Lösung des Alkalimetallalkoholats oder -phenolats, gelöst in einem der vorstehend genannten organischen Lösungsmittel, zweckmäßigerweise im gleichen Lösungsmittel, in welchem das 2,4-Dinitrochlorbenzol gelöst zur Anwendung gelangt, innerhalb des weiter oben genannten Temperaturbreichs, vorzugsweise zwischen 0 und 10 °C, zuzutropfen. Die beiden Reaktionskomponenten werden in äquimolaren Mengen eingesetzt.

Bei der nächsten Stufe (Reduktion der beiden Nitrogruppen in 2,4-Stellung des Benzolkerns) werden als Edelmetallkatalysatoren der 8. Nebengruppe des Periodischen Systems beispielsweise Platin, Platinoxid oder Palladium, jeweils auf geeignetem Trägermaterial, wie Kohle oder Bariumsulfat, angewandt, wobei der Katalysator zweckmäßigerweise in einer Menge von 0,5-10 g, vorzugsweise von 4-6 g pro Mol 2,4-Dinitrophenylether, eingesetzt wird. Als hierbei ebenfalls in katalytischen Mengen eingesetze organische Stickstoffbasen kommen beispielsweise aromatische Basen, wie Pyridin oder

Chinolin, oder heterocyclische Basen, wie Morpholin, oder Trialkyl $C_1$-$C_4$ amine, wie beispielsweise Triethylamin oder Tri-n-butylamin, oder Dialkyl-$C_1$-$C_4$ amine, wie beispielsweise Diisopropylamin, oder ferner Trialkylolamine mit 1-4 Kohlenstoffatomen im Alkylteil, wie z. B. Triäthanolamin, in Betracht, wobei die organische Stickstoffbase zweckmäßigerweise in einer Menge von 1 bis 3 Gew.-%, vorzugsweise von 1 bis 1,5 Gew.-%, bezogen auf eingesetzten 2,4-Dinitrophenylether angewandt wird. Im übrigen kommen bei dieser Reaktionsstufe dieselben polaren organischen Lösungsmittel zur Anwendung wie bei der ersten Reaktionsstufe (Chloraustausch).

Die in dritter Reaktionsstufe erfolgende selektive Acylierung der in 4-Stellung des Benzolkerns befindlichen Aminogruppe wird ebenfalls in den gleichen polaren organischen Lösungsmitteln, wie sie bei der ersten Stufe (Chloraustausch) zur Anwendung kommen, durchgeführt. Als Acylierungsmittel kommen Carbonsäureanhydride bzw. Carbonsäurehalogenide der allgemeinen Formeln

$$(\text{Alkyl } C_1\text{-}C_4 \text{ CO})_2O \text{ bzw.}$$

$$\text{Alkyl } C_1\text{-}C_4\text{—CO—Halogen}$$

worin Halogen vorzugsweise ein Chloratom bedeutet, in Frage.

Ebenso wird die vierte Reaktionsstufe (selektive Alkylierung der in 2-Stellung des Benzolkerns befindlichen Aminogruppe) in den polaren organischen Lösungsmitteln durchgeführt, die bei der ersten Reaktionsstufe angewandt werden (siehe weiter oben).

An hierbei zur Anwendung gelangenden Edelmetallkatalysatoren und in katalytischen Mengen eingesetzten organischen Stickstoffbasen kommen die gleichen wie bei der vorstehend beschriebenen Reduzierung der 2,4-Dinitrophenylether angegebenen Katalysatoren und organischen Stickstoffbasen in Betracht. Die Edelmetallkatalysatoren werden hierbei zweckmäßigerweise in einer Menge von 1-10 g, vorzugsweise 1-5 g pro Mol 4-Acylamino-2-amino-phenylether eingesetzt. Die organischen Basen werden hierbei zweckmäßigerweise in einer Menge von 2 bis 10 Gew.-%, vorzugsweise 4 bis 6 Gew.-%, bezogen auf eingesetzten 4-Acylamino-2-amino-phenylether angewandt.

Der Aldehyd wird zweckmäßigerweise in einem Überschuß von 20-80 Molprozent, vorzugsweise von 25 Molprozent, bezogen auf die Verbindung der genannten allgemeinen Formel (4), angewandt.

4-Acylamino-2-alkylamino-phenylether sind wertvolle Vorprodukte zur Herstellung von Dispersionsfarbstoffen (Belgische Patentschrift 634 032).

## Beispiel 1

Zu einer Lösung von 98 g (1 Mol) Natriummethylglycolat in 407 g Methylglycol werden 202,6 g (1 Mol) 2,4-Dinitrochlorbenzol in 482 g Methylglycol bei 0-10 °C innerhalb einer halben Stunde zugetropft. Anschließend wird eine Stunde nachgerührt, von ausgefallenem Natriumchlorid abfiltriert und das Filtrat mit 12,6 g Triethylamin und 5 g Katalysator (5 % Palladium auf Kohle) versetzt. Die Mischung wird bei 50-60 °C und einem Wasserstoffdruck von 10-30 bar hydriert. Der Katalysator wird abfiltriert und der Gehalt der Lösung an 4-Diamino-β-methoxyethoxybenzol durch Nitrittitration ermittelt (Ausbeute 94 % der Theorie). Methylglycol und Wasser werden unter vermindertem Druck (22 mm) destillativ entfernt und der Rückstand in 735 g Methanol aufgenommen. Daraufhin wird die Lösung mit 20,2 g (0,5 Mol) Magnesiumoxid versetzt. Dann werden bei 0-15 °C 93,9 g (0,92 Mol) Essigsäureanhydrid innerhalb von zweieinhalb Stunden zugetropft, wobei man in hoher Selektivität 4-Acetylamino-2-amino-β-methoxyethoxybenzol erhält. Produktverteilung ermittelt durch HPLC-Analyse (« HPLC » = high pressure liquid chromatography) :

| | |
|---|---|
| 2,4-Diamino-β-methoxyethoxybenzol | 4 % |
| 4-Acetylamino-2-amino-β-methoxyethoxybenzol | 89 % |
| 2,4-Diacetylamino-β-methoxyethoxybenzol | 7 % |

665,7 g der Mischung (≙ 0,5 Mol 4-Acetylamino-2-amino-β-methoxyethoxybenzol), werden mit 12,5 g Aktivkohle und 12,5 g Kieselgur geklärt und mit 6,3 g Triethylamin und 2,5 g Katalysator (5 % Palladium auf Kohle) versetzt. Die Mischung wird mit 26,6 g (0,6 Mol) Acetaldehyd versetzt und bei 50-60 °C und einem Wasserstoffdruck von 40-80 bar hydriert. Der Katalysator wird abfiltriert, Methanol bei Atmosphärendruck abdestilliert und das Produkt durch Fällung mit 200 g Wasser isoliert.

Selektivität der Alkylierung (HPLC-Analyse) :

| | |
|---|---|
| 4-Acetylamino-2-amino-β-methoxyethoxybenzol | 4,6 % |
| 4-Acetylamino-2-ethylamino-β-methoxyethoxybenzol | 93 % |
| 4-Acetylamino-2-diethylamino-β-methoxyethoxybenzol | 2,4 % |

Die Ausbeute an 4-Acetylamino-2-ethylamino-β-methoxyethoxybenzol beträgt 72 % der Theorie, bezogen auf eingesetztes 2,4-Dinitrochlorbenzol. Fp. 72-73 °C.

Verwendet man anstelle von 12,6 g Triethylamin 12,6 g Diisopropylamin und verfährt im übrigen wie

vorstehend beschrieben, so gelangt man hinsichtlich Ausbeute und Zusammensetzung des Endprodukts zum gleichen Ergebnis.

Verwendet man anstelle von 12,6 g Triethylamin 12,6 g Tri-n-butylamin und verfährt im übrigen wie vorstehend beschrieben, so gelangt man hinsichtlich Ausbeute und Zusammensetzung des Endprodukts zum gleichen Ergebnis.

Beispiel 2

Eine Lösung von 119 g (0,5 Mol) 2-Amino-4-propionylamino-β-methoxyethoxybenzol (hergestellt analog Beispiel 1) in 395 g Methanol wird mit 6,3 g Aktivkohle und 6,3 g Kieselgur geklärt. Darauf wird das Filtrat mit 6,3 g Triethylamin und 2,5 g Katalysator (5 % Palladium auf Kohle) versetzt. Anschließend wird in Gegenwart von 33,3 g (0,75 Mol) Acetaldehyd inner-halb von zwei Studen bei 50-60 °C und einem Wasserstoffdruck von 40-80 bar hydriert. Nach Abtrennung des Katalysators von der Reaktionslösung unter Stickstoff werden Methanol und Wasser abdistilliert. Dann wird das Produkt durch Zugabe von 180 g Wasser gefällt, abgesaugt und mit 100 g 20 %iger Kochsalzlösung gewaschen. Die Ausbeute an 2-Ethylamino-4-propionylamino-β-methoxyethoxybenzol beträgt 84 % der Theorie, bezogen auf eingesetztes 2-Amino-4-propionylamino-β-methoxyethoxybenzol. Schmelzpunkt einer getrockneten Probe : 78-80 °C.

Selektivität der Alkylierung (HPLC-Analyse) :

| | |
|---|---|
| 2-Amino-4-propionylamino-β-methoxyethoxybenzol | 4 % |
| 2-Ethylamino-4-propionylamino-β-methoxyethoxybenzol | 93,5 % |
| 2-Diethylamino-4-propionylamino-β-methoxyethoxybenzol | 2,5 % |

Beispiel 3

Eine Lösung von 90 g (0,5 Mol) 4-Acetylamino-2-amino-anisol (hergestellt analog Beispiel 1) in 395 g Methanol wird versetzt mit 6,3 g Triethylamin und 2,5 g Katalysator (5 % Palladium auf Kohle). Die Mischung wird in Gegenwart von 26,4 g (0,6 Mol) Acetaldehyd bei einem Wasserstoffdruck von 40-80 bar und einer Temperatur von 50-60 °C innerhalb von zwei Stunden hydriert. Nach Abfiltrieren des Katalysators wird das Methanol abdistilliert und das Produkt durch Zugabe von 200 g Wasser gefällt. Die Ausbeute an 4-Acetylamino-2-ethylaminoanisol beträgt 86 % der Theorie. Fp. 94-102 °C.

Selektivität der Alkylierung (HPLC-Analyse) :

| | |
|---|---|
| 4-Acetylamino-2-amino-anisol | 2 % |
| 4-Acetylamino-2-ethylamino-anisol | 96 % |
| 4-Acetylamino-2-diethylamino-anisol | 2 % |

Beispiel 4

Eine Lösung von 121 g (0,5 Mol) 4-Acetylamino-2-amino-diphenylether (hergestellt analog Beispiel 1) in 395 g Methanol wird versetzt mit 6,3 g Triethylamin und 2,5 g Katalysator (5 % Palladium auf Kohle). Die Mischung wird in Gegenwart von 28,6 g (0,65 Mol) Acetaldehyd eine Stunde bei 50-60 °C und einem Wasserstoffdruck von 40-80 bar hydriert. Nach Abfiltrieren des Katalysators wird das Lösungsmittel destillativ entfernt und das Produkt durch Zugabe von 200 g Wasser gefällt.

Die Ausbeute an 4-Acetylamino-2-ethylaminodiphenylether beträgt 88 % der Theorie, Fp. 109-110 °C.

Selektivität der Alkylierung (HPLC-Analyse) :

| | |
|---|---|
| 4-Acetylamino-2-amino-diphenylether | 5 % |
| 4-Acetylamino-2-ethylamino-diphenylether | 89 % |
| 4-Acetylamino-2-diethylamino-diphenylether | 6 % |

Beispiel 5 (Vergleichsbeispiel hinsichtlich reduktiver Alkylierung)

Eine Lösung von 112 g (0,5 Mol) 4-Acetylamino-2-amino-β-methoxyethoxybenzol (hergestellt gemäß Beispiel 1) in 395 g Methanol wird in Gegenwart von 4,3 g 60 %igem Raney-Nickel und 28,6 g (0,65 Mol) Acetaldehyd bei 60-70 °C und einem Wasserstoffdruck von 40-80 bar hydriert. Nach Abfiltrieren des Katalysators wird das Produkt durch Zugabe von 200 g Wasser gefällt.

Die Ausbeute an 4-Acetylamino-2-ethylamino-β-methoxyethoxybenzol beträgt 71 % der Theorie.

Selektivität der Reaktion (HPLC-Analyse) :

| | |
|---|---|
| 4-Acetylamino-2-amino-β-methoxyethoxybenzol | 15 % |
| 4-Acetylamino-2-ethylamino-β-methoxyethoxybenzol | 77 % |
| 4-Acetylamino-2-diethylamino-β-methoxyethoxybenzol | 8 % |

Beispiel 6 (Vergleichsbeispiel hinsichtlich reduktiver Alkylierung)

Eine Lösung von 112 g (0,5 Mol) 4-Acetylamino-2-amino-β-methoxyethoxybenzol in 395 g Methanol wird in Gegenwart von 6,3 g Triethylamin, 5 g Nickel-Kieselgur-Katalysator (RCH 55/5 Hersteller Ruhrchemie) und 33 g (0,75 Mol) Acetaldehyd eineinhalb Stunden bei 60-85 °C und einem Wasserstoffdruck von 40-80 bar hydriert. Nach Abfiltrieren des Katalysators wird das Lösungsmittel abdestilliert und das Produkt durch Zugabe von 200 g Wasser ausgefällt. Die Ausbeute an 4-Acetylamino-2-ethylamino-β-methoxyethoxybenzol beträgt 11 % der Theorie.

Selektivität (HPLC-Analyse) :

| | |
|---|---|
| 4-Acetylamino-2-amino-β-methoxyethoxybenzol | 68 % |
| 4-Acetylamino-2-ethylamino-β-methoxyethoxybenzol | 14 % |
| 4-Acetylamino-2-diethylamino-β-methoxyethoxybenzol | 18 % |

Beispiel 7 (Vergleichsbeispiel)

213 g geschmolzenes 2,4-Dinitrochlorbenzol werden in 632 g Methanol eingebracht und unter Rühren gelöst. Zu der gerührten Lösung werden 42 g festes Natriumhydroxid in Schuppenform so zugegeben, daß die Reaktionslösung zum schwachen Sieden am Rückfluß erhitzt wird. Nach Zusatz der gesamten Natriumhydroxidmenge wird 1 Stunde am Rückfluß erhitzt. Anschließend läßt man abkühlen (vgl. Reaktionsstufe 1 von Beispiel 1 der Europäischen Patentschrift 0 011 048).

Die so erhaltene Suspension (enthaltend 2,4-Dinitroanisol) wird an 5 % Palladium/Kohle bei 60 °C hydriert, wobei die Ausbeute an 2,4-Diaminoanisol 95,2 % der Theorie entspricht.

Beispiel 8 (Vergleichsbeispiel)

Eine analog Beispiel 1 durch Zutropfen einer methanolischen Lösung von 2,4-Dinitrochlorbenzol zu einer methanolischen Lösung von Natriummethylat innerhalb einer halben Stunde bei 0-10 °C und 1-stündiges Nachrühren hergestellte Suspension enthaltend 2,4-Dinitroanisol wird mit 45 g Wasser versetzt (d. h. mit der Wassermenge, die gemäß Reaktionsstufe 1 von Beispiel 1 der Europäischen Patentschrift 0 011 048 bei Verwendung des dort alternativ angegebenen Zusatzes von 50 %iger wäßriger Natronlauge im Reaktionsgemisch vorhanden wäre). Nach der analog Beispiel 1 durchgeführten katalytischen Reduktion erhält man eine Ausbeute an 2,4-Diaminoanisol von nur 87,6 % der Theorie.

Beispiel 9

Zu einer Suspension von 202,5 g 2,4-Dinitrochlorbenzol in 440 Teilen Methanol läßt man bei Raumtemperatur innerhalb von 60 Minuten 189,1 g einer 30 %igen Lösung von Natriummethylat in Methanol laufen, wobei die Temperatur auf 55-60 °C ansteigt. Nach Zugabe von 160 Teilen Methanol und 5 Teilen Triethylamin wird an 5 % Palladium/Kohle bei 60 °C hydriert. Die Ausbeute an 2,4-Diaminoanisol beträgt 98,0 % der Theorie.

**Patentansprüche**

1. Verfahren zur Herstellung von 4-Acylamino-2-alkylaminophenylethern der allgemeinen Formel (1)

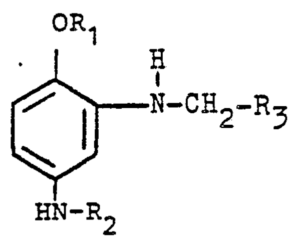

(1)

in welcher $R_1$ eine Alkyl $C_1$-$C_4$ oder Alkoxy $C_1$-$C_4$ alkyl $C_1$-$C_4$ Gruppe oder eine Phenylgruppe, die durch Chlor- oder Bromatome, Alkyl $C_1$-$C_4$, Alkoxy $C_1$-$C_4$, Trifluormethyl-, Carbonsäureamid-, —NH—Alkyl $C_1$-$C_4$, —N(Alkyl $C_1$-$C_4$)$_2$, —$SO_2$—$NH_2$—, —$SO_2$—NH—Alkyl $C_1$-$C_4$, —$SO_2$—N(Alkyl $C_1$-$C_4$)$_2$—, Acetyl-, Benzoylamino- oder —NH—CO—Alkyl $C_1$-$C_4$-Gruppe substituiert sein kann, $R_2$ eine

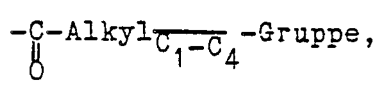

und $R_3$ eine Alkyl-$C_1$-$C_4$-Gruppe bedeuten, dadurch gekennzeichnet, daß man wasserfrei in einem polaren organischen Lösungsmittel 2,4-Dinitrochlorbenzol mit einem Alkalimetallalkoholat oder -phenolat der allgemeinen Formel (2)

$$R_1—OMe \qquad (2)$$

worin $R_1$ die vorstehend genannte Bedeutung hat und Me ein Alkalimetallatom darstellt, bei Temperaturen von — 30 °C bis 100 °C, zum 2,4-Dinitrophenyläther der allgemeinen Formel (3)

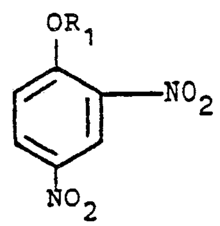

(3)

worin $R_1$ die vorstehend genannte Bedeutung hat, umsetzt, diesen nach oder vorzugsweise ohne Zwischenisolierung in einem polaren organischen Lösungsmittel, vorzugsweise im gleichen Lösungsmittel, in welchem der Chloraustausch erfolgte, in einer Wasserstoffatmosphäre in Gegenwart eines Edelmetallkatalysators der 8. Nebengruppe des Periodischen Systems (Ruthenium bis Platin) und einer katalytischen Menge einer organischen Stickstoffbase bei Temperaturen von 50 bis 120 °C zum entsprechenden 2,4-Diaminophenylether reduziert, diesen nach oder vorzugsweise ohne Zwischenisolierung in einem polaren organischen Lösungsmittel bei Temperaturen von — 30 bis + 90 °C selektiv acyliert zum entsprechenden 4-Acylamino-2-amino-phenylether der allgemeinen Formel (4)

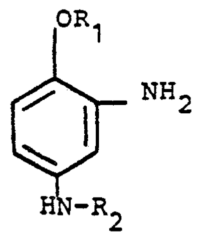

(4)

in welcher $R_1$ und $R_2$ die vorstehend genannten Bedeutungen haben und diesen nach oder vorzugsweise ohne Zwischenisolierung in einem polaren organischen Lösungsmittel mit einem Aldehyd der allgemeinen Formel (5)

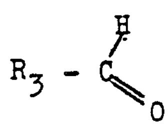

(5)

in welcher $R_3$ die vorstehend genannte Bedeutung hat, in einer Wasserstoffatmosphäre in Gegenwart eines Edelmetallkatalysators der 8. Nebengruppe des Periodischen Systems (Ruthenium bis Platin) und einer katalytischen Menge einer organischen Stickstoffbase bei Temperaturen von 30 bis 120 °C, selektiv alkyliert.

2. Verfahren nach Anspruch 1, dadurch gekennzeichnet, daß man die Reaktionen der vier Verfahrensstufen in einem geradkettigen oder verzweigten Alkanol von 1-4 Kohlenstoffatomen oder in einem Glykol oder Glykoläther als polarem organischen Lösungsmittel durchführt.

3. Verfahren nach Anspruch 1 oder 2, dadurch gekennzeichnet, daß man die Reduktion und die selektive Alkylierung in Gegenwart von Pyridin, Chinolin, Morpholin, einem Trialkyl $C_1$-$C_4$ amin, einem Dialkyl $C_1$-$C_4$ amin oder einem Trialkylolamin mit 1-4 Kohlenstoffatomen im Alkylrest als organischer stickstoffhaltiger Base durchführt.

**Claims**

1. A process for the preparation of 4-acylamino-2-alkylamino-phenyl ethers of the general formula (1)

(See formula page 8)

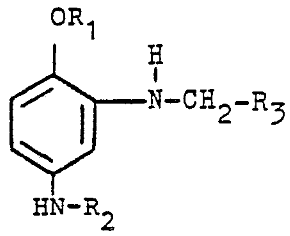

(1)

in which $R_1$ stands for an alkyl ($C_1$-$C_4$) or alkoxy ($C_1$-$C_4$)-alkyl ($C_1$-$C_4$) group or a phenyl group which can be substituted by chlorine or bromine atoms, alkyl ($C_1$-$C_4$), alkoxy ($C_1$-$C_4$), trifluoromethyl, carboxylic acid amide, —NH—alkyl ($C_1$-$C_4$), —N(alkyl) $(C_1$-$C_4)_2$, —$SO_2$—$NH_2$, —$SO_2$—NH—alkyl ($C_1$-$C_4$), —$SO_2$—N(alkyl $(C_1$-$C_4))_2$, acetyl, benzoylamino or —NH—CO—alkyl ($C_1$-$C_4$) group, $R_2$ stands for a

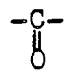 alkyl ($C_1$-$C_4$) group

$R_3$ stands for an alkyl ($C_1$-$C_4$) group, wherein 2,4-dinitrochlorobenzene is reacted anhydrously in a polar organic solvent with an alkali metal alcoholate or phenolate of the general formula (2)

$$R_1\text{—OMe} \qquad (2)$$

where $R_1$ has the meaning cited above and Me represents an alkali metal atom, at temperatures of — 30 °C to 100 °C, to produce the 2,4-dinitrophenyl ether of the general formula (3)

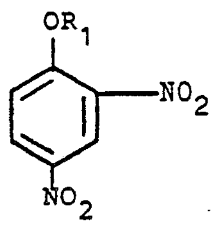

(3)

where $R_1$ has the meaning cited above, the latter compound, after or preferably without intermediate isolation, is reduced to the corresponding 2,4-diaminophenyl ether in a polar organic solvent, preferably in the same solvent in which the chlorine exchange was carried out, in a hydrogen atmosphere in the presence of a precious-metal catalyst of group VIII of the Periodic Table (ruthenium to platinum) and a catalytic quantity of an organic nitrogen base at temperatures of 50 to 120 °C, the latter compound, after or preferably without intermediate isolation, is selectively acylated in a polar organic solvent at temperatures of — 30 to + 90 °C to produce the correponding 4-acylamino-2-amino-phenyl ether of the general formula (4)

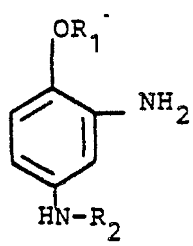

(4)

in which $R_1$ and $R_2$ have the meanings cited above, and the latter compound, after or preferably without intermediate isolation, is selectively alkylated in a polar organic solvent with an aldehyde of the general formula (5)

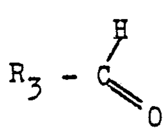

(5)

in which $R_3$ has the meaning cited above, in a hydrogen atmosphere in the presence of a precious-metal catalyst of group VIII of the Periodic Table (ruthenium to platinum) and a catalytic quantity of an organic nitrogen base at temperatures of 30 to 120 °C.

2. The process as claimed in claim 1, wherein the reactions of the four process stages are carried out in a straight-chain or branched alkanol containing 1-4 carbon atoms or in a glycol or glycol ether as the polar organic solvent.

3. The process as claimed in claim 1 or 2, wherein the reduction and the selective alkylation are carried out in the presence of pyridine, quinoline, morpholine, a trialkyl ($C_1$-$C_4$) amine, a dialkyl ($C_1$-$C_4$) amine or a trialkylolamine containing 1-4 carbon atoms in the alkyl radical as an organic nitrogen-containing base.

**Revendications**

1. Procédé de préparation d'éthers alkylamino-2 acylamino-4 phényliques répondant à la formule générale (1) :

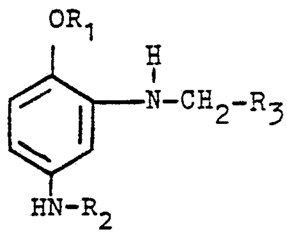

(1)

dans laquelle $R_1$ représente un alkyle $C_1$-$C_4$, un alcoxy $C_1$-$C_4$ alkyle $C_1$-$C_4$ ou un radical phényle éventuellement porteur d'atomes de chlore ou de brome, d'un alkyle $C_1$-$C_4$, d'un alcoxy $C_1$-$C_4$, d'un trifluorométhyle, d'un carbamoyle, d'un radical —NH—alkyl $C_1$-$C_4$, d'un radical —N(alkyl $C_1$-$C_4$)$_2$, d'un radical —$SO_2$—$NH_2$, d'un radical —$SO_2$—NH—alkyl $C_1$-$C_4$, d'un radical —$SO_2$—N(alkyl-$C_1$-$C_4$)$_2$, d'un acétyle, d'un benzolylamino ou d'un radical —NH—CO—alkyl $C_1$-$C_4$, $R_2$ représente un radical —C—alkyl $C_1$-$C_4$

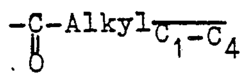

et $R_3$ représente un radical alkyle en $C_1$-$C_4$, procédé caractérisé en ce qu'on fait réagir, en l'absence d'eau, dans un solvant organique polaire, le dinitro-2,4 chlorobenzène avec un alcoolate ou un phénolate de métal alcalin répondant à la formule générale (2) :

$$R_1\text{—OMe} \qquad (2)$$

dans laquelle $R_1$ a la signification indiquée ci-dessus et Me représente un atome de métal alcalin, à des températures de — 30 à 100 °C, de manière à obtenir l'éther dinitro-2,4 phénylique répondant à la formule générale (3) :

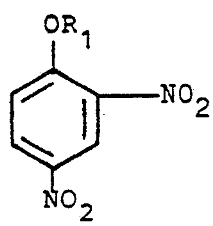

(3)

dans laquelle $R_1$ a la signification indiquée ci-dessus, on réduit ce composé, après l'avoir ou, mieux, sans l'avoir isolé intermédiairement, dans un solvant organique polaire, de préférence dans le même solvant que celui dans lequel on a effectué l'échange de chlore, sous atmosphère d'hydrogène, en présence d'un catalyseur à base d'un métal noble du huitième sous-groupe de la classification périodique (du ruthénium au platine) et d'une quantité catalytique d'une base azotée organique, à des températures de 50 à 120 °C, réduction qui donne naissance à l'éther diamino-2,4 phénylique correspondant, on acyle sélectivement ce dernier, après l'avoir ou, mieux, sans l'avoir isolé intermédiairement, dans un solvant organique polaire, à des températures de — 30 à + 90 °C, de manière à le convertir en l'éther amino-2 acylamino-4 phénylique correspondant qui répond à la formule générale (4) :

(Voir formule page 10)

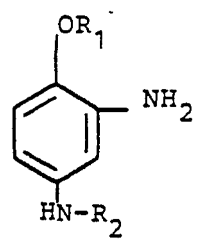

(4)

dans laquelle $R_1$ et $R_2$ ont les significations indiquées ci-dessus, et on alkyle sélectivement celui-ci, après l'avoir ou, mieux, sans l'avoir isolé intermédiairement, dans un solvant organique polaire, au moyen d'un aldéhyde répondant à la formule générale (5) :

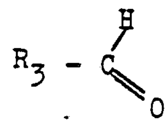

(5)

dans laquelle $R_3$ a la signification indiquée ci-dessus, sous atmosphère d'hydrogène, en présence d'un catalyseur à base d'un métal noble du huitième sous-groupe de la classification périodique (du ruthénium au platine) et d'une quantité catalytique d'une base azotée organique, à des températures de 30 à 120 °C.

2. Procédé selon la revendication 1 caractérisé en ce qu'on effectue les réactions des quatre étapes opératoires dans un alcanol linéaire ou ramifié qui contient de 1 à 4 atomes de carbone ou dans un glycol ou dans un éther de glycol, comme solvant organique polaire.

3. Procédé selon l'une des revendications 1 et 2, caractérisé en ce qu'on effectue la réduction et l'alkylation sélective en présence de pyridine, de quinoléine, de morpholine, d'une trialkyl $C_1$-$C_4$ amine, d'une dialkyl $C_1$-$C_4$ amine ou d'une trialkylol-amine dont chacun des alkyles contient de 1 à 4 atomes de carbone, comme base azotée organique.